# FASCICULE DE BREVET EUROPEEN

(11) **EP 2 730 629 B1**
(45) Date de publication et mention de la délivrance du brevet: **14.10.2015**
(21) Numéro de dépôt: 13170897.6
(22) Date de dépôt: 06.06.2013
(51) Int. Cl.: C09J 7/04, C09J 183/06, A61L 15/26, B32B 27/12

(54) **Ruban adhésif en silicone destiné à des applications médicales**
Silikonklebeband für medizinische Anwendungen
Silicone adhesive tape for medical applications

(30) Priorité: 13.11.2012 US 201261725681 P
(43) Date de publication de la demande: 14.05.2014
(73) Titulaire: Zodiac Coating, 78370 Plaisir (FR)
(72) Inventeur: Guillo, Jean-Roger, 38300 SEREZIN DE LA TOUR (FR); Lecoeuvre, Jean-François, 42290 SORBIERS (FR); Guillermin, Mélanie, 38230 Charvieu Chavagneux (FR)
(74) Mandataire: Zimmermann, Alain

(56) Documents cités:
- WO-A2-2010/121033
- FR-A1- 2 971 971
- US-A1- 2002 026 166
- US-A1- 2009 053 441

## Description

La présente invention concerne les rubans d'intissé notamment des rubans adhésifs simple face, des patchs ou autocollants de maintien de dispositifs médicaux tels que aiguilles, capteurs, des pansements adhésifs ou en particulier destinés au contact atraumatique de la peau saine et de la plaie.

Habituellement les bandes adhésives destinées à la confection de pansement et au maintien de dispositifs médicaux sont constituées de substrat plan, comme par exemple des feuilles d'intissé, des tissus, des films ou des membranes enduits d'un coté soit d'un polymère adhésif acrylique soit de formulations à base de caoutchouc comprenant des résines, plastifiants ou polymères pégueux.

Ces compositions sont généralement à base de polymères de haut poids moléculaire, et la force d'adhésion sur la peau dépend de la composition et de la nature des résines tackifiantes utilisées.

Ces polymères à haut poids moléculaire confèrent à la formulation des viscosités élevées, en général supérieures à plusieurs milliers de mPa.s, qui nécessitent un processus adapté pour enduire le substrat de quelques dizaines ou centaines de g/m², qui constitue la couche généralement nécessaire pour obtenir une adhésion suffisante au substrat et à la peau.

Afin d'enduire de façon satisfaisante le substrat comme expliqué précédemment, il est généralement procédé à une dissolution de la formulation dans un solvant, ou en émulsion aqueuse, et ensuite cette dissolution est enduite par des procédés conventionnels d'enduction, par exemple à l'aide d'une racle ou des cylindres, avant de procéder à l'évaporation du solvant au travers du passage dans une étuve à air chaud, afin d'obtenir une couche sèche de formulation appropriée.

Dans certains cas il peut être utilisé des polymères hotmelt sans solvant. Dans ce cas la mise en oeuvre de l'enduction doit être réalisée pendant la phase de fusion du polymère, avant refroidissement et solidification de la couche de polymère sur le substrat.

Usuellement, la haute viscosité du compound en dissolution ou en émulsion ou à l'état fondu nécessite un ajustement de la viscosité de la forme liquide afin de régler l'enduction de la surface du substrat au contact de la peau.

De plus, d'un point de vue fonctionnel, ces compositions de polymères contribuent à l'apparition de résidus polymériques collés sur la peau lors du retrait de l'adhésif. Ainsi, l'utilisation de ce type de polymères/ compositions, cette très forte adhésion, et la faible biocompatibilité (irritabilité, sensibilité de la peau) de ces ingrédients provoquent une adhésion traumatique du ruban sur la peau, notamment sur les peaux fragile telles que celle des personnes âgées, des enfants, des prématurés, des brulés.

Très récemment, il a été développé et sont apparus sur le marché quelques rubans à adhésion « légère » à base de polymères de silicone PSA (« Pressure Sensitive Adhesives»). Ces compositions sont à base de caoutchouc de silicone réticulé, partiellement ou totalement, comprenant des résines de silicone à haut pouvoir tackifiant qui procure au ruban une adhésion contact significative sur la peau.

Cependant, de la même façon, la haute viscosité de ces polymères impose une mise en dissolution dans un solvant afin d'ajuster la rhéologie de la pate liquide pour leur enduction sur un substrat plan.

En général, la polarité des solvants utilisés et la faible viscosité de la dissolution sont telles que les fibres du substrat sont totalement imprégnées et se laissent traverser par la dissolution pour exsuder sur la face externe du substrat, qui devient alors pégueuse et impropre à un enroulement du ruban sur lui-même, et à une utilisation ultérieure externe sur un patient.

Par ailleurs la nature des solvants utilisés engendre, lors de leur utilisation, des risques importants pour l'hygiène et la santé du personnel de fabrication et pour l'environnement, ainsi que des coûts importants.

Afin d'éviter que la composition tackifiante traverse le ruban vers sa face externe, les fournisseurs d'intissé ou les fabricants de ruban ont procédé au laminage, sur la face interne de l'intissé, d'une membrane ou d'un film de polymère généralement à base de polyuréthane ou de polyoléfine avant d'enduire ladite membrane de la composition tackifiante.

Cependant, cette opération représente un coût significatif et des risques importants pour l'obtention d'une adhérence forte de la composition tackifiante sur ce film.

Ce risque d'adhésion faible constitue à son tour un risque important de délamination de la couche adhésive et du substrat lors du retrait du ruban de la peau du patient, avec les inconvénients de résidus et de traumatisme sur la peau décrits précédemment.

Ainsi, les solutions de l'art antérieur ne donnent pas entièrement satisfaction.

Un but de la présente invention est donc de résoudre les problèmes cités précédemment, à l'aide d'une solution simple à fabriquer, peu coûteuse, facile à utiliser et optimisée en termes d'efficacité.

Ainsi, la présente invention a pour objet un ruban adhésif en silicone destiné à des applications médicales, comprenant un matériau en feuille à base de fibres intissées, enduit :
- sur une face interne destinée à être au contact de la peau, d'une membrane barrière adhérente au matériau en feuille, à base de polymère de silicone réticulé et d'une couche de gel adhésif de silicone atraumatique et biocompatible, et
- sur une face externe, d'une couche d'un polymère anti-adhérent et déperlant à l'eau à base d'un polymère de fluorosilicone ou de fluorocarbone.

La présente invention se rapporte ainsi à une bande de matériaux à base de nappe de fibres intissées ne présentant pas de risque de transfert de silicone gel de la face intérieure au contact de la peau vers la face externe, et procurant au patient un confort de port, une aisance d'enroulage et de déroulage, associée à une aisance de déchirage transversal manuel permettant de découper un morceau de la taille désirée sans instrument.

Selon des modes de réalisation préférés, le dispositif conforme à la présente invention comprend l'une au moins des caractéristiques suivantes :
- le matériau en feuille est composé d'une ou deux nappes d'un mélange de fibres à faible résistance à la déchirure et aisément déchirable à la main, fabriquée(s) à partir d'un mélange de fibres courtes de cellulose et de fibres de polyéthylènetéréphtalate de longueur moyenne, collées entre elles par un calandrage sous pression à chaud et/ou par un agent chimique de liage en émulsion aqueuse ;
- le ratio de partage entre les fibres varie de 10 à 40% de PET pour 90 à 60 % de cellulose, le poids de la nappe intissée varie de l'ordre de 30 à 70 g/m2 pour une épaisseur de 0,10 à 0,30 mm, les fibres de cellulose présentent une longueur de l'ordre de 1mm à 20mm, et les fibres de PET présentent des structures de fibre cylindrique de masse linéique comprise entre 0.1dtex et 5dtex, et de longueur de l'ordre de 2mm à 20mm ;
- la nappe intissée présente une structure de surface comportant des petits plis de matière en surabondance dans le sens transversal de façon à conférer au matériau des propriétés de déformation sous faible contrainte d'allongement dans le sens longitudinal ;
- le ruban comporte également une couche d'un gel de silicone réticulé adhésif atraumatique et biocompatible pour la peau adhérent par réaction de copolymérisation sur une membrane en silicone réticulée formant barrière sur la face interne de la nappe intissée ;
- le ruban comporte en outre une fine membrane barrière continue ou une couche discontinue en polymère réticulé de silicone contrecollée aux fibres de la face interne de la nappe intissée ; cette membrane a pour fonction de constituer un primaire d'adhésion de forte adhérence aux fibres et au gel de silicone atraumatique qui sera ensuite enduit par-dessus, de constituer une couche barrière pour éviter la diffusion du gel de silicone adhésif au travers des fibres et de constituer une barrière chimique entre le gel de silicone et les composants de l'intissé susceptibles d'être des inhibiteurs à la réaction de polymérisation du gel à base de catalyseur de platine ;
- le ruban est perforé de petits orifices de façon à lui procurer une grande perméabilité à la vapeur issue de l'exsudation de la peau et afin d'éviter une macération de la peau humide ;
- la feuille de fibres intissées est perforée d'orifices de diamètre de 0,05 à 0,5 mm de manière à procurer au matériau final un aspect de structure textile tissé ou tricoté afin d'améliorer la perméabilité à la vapeur d'eau issue de l'exsudation de la peau, d'améliorer la déchirabilité, et/ou de procurer à la surface externe du matériau des propriétés de faible friction et de grand confort de port, au contact avec tout textile ou vêtement, et/ou afin de procurer pour le patient un grand confort de port, au contact de zone de la peau susceptible de se déformer, de se plier, ou de comporter des zones creuses et des zones à relief ;
- les bords du ruban sont découpés sur toute leur longueur selon un motif géométrique répétitif, tel que dents triangulaires, des demi disques, ou des indentations aléatoires, afin de procurer au ruban une facilité de déchirure manuelle transversale ;
- la perméabilité à la vapeur d'eau de la couche de gel adhésif de silicone est comprise entre environ 500 à 2000 g/m²/24h, mesurée à l'eau selon la méthode de la coupe inversée (norme EN 13 726-2), et de préférence d'environ 1000 g/m²/24h ; et
- les fibres intissées sont teintes, et/ou l'agent chimique de liaison des fibres est pigmenté et enduit d'une couche barrière de silicone réticulé et d'une couche d'un adhésif de gel de silicone réticulé dans lesquelles les polymères de silicone sont pigmentés.

L'invention va maintenant être décrite plus en détail en référence à des modes de réalisation particuliers donnés à titre d'illustration uniquement et représentés sur les figures annexées dans lesquelles :
- La figure 1 est une vue en coupe d'un ruban adhésif conforme à la présente invention ;
- Les figures 2 à 4 sont des vue de dessus d'une feuille déchirable facilement à base de fibres intissées appartenant au ruban adhésif ;
- La figure 5 est une de dessus d'une variante de réalisation des figures 2 à 4 ; et
- La figure 6 est une vue en coupe d'un ruban adhésif conforme à la variante de réalisation de la figure 5.

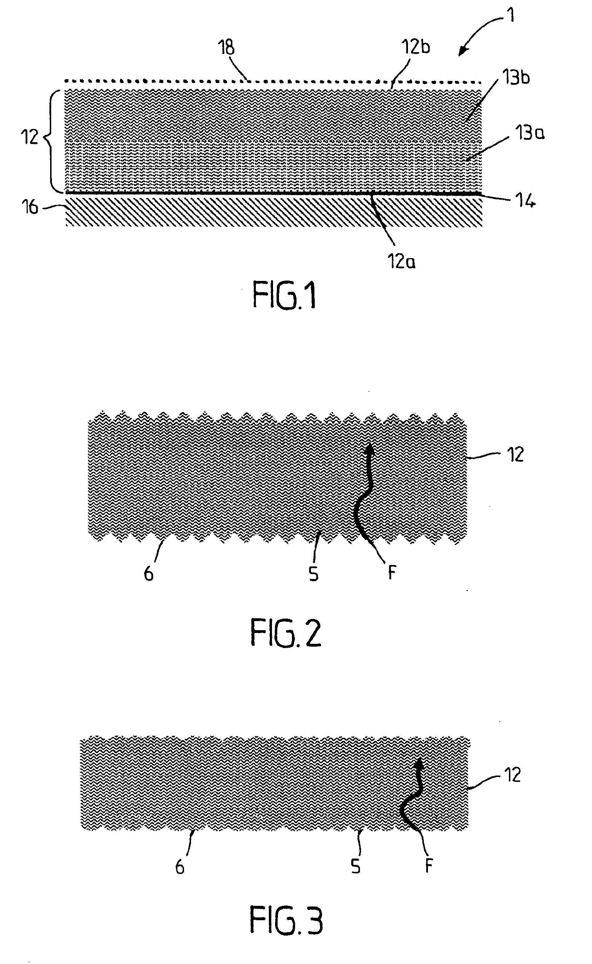

La figure 1 représente un ruban adhésif 1 destiné à des applications médicales, par exemple en vue de la réalisation d'un pansement, comportant un matériau en feuille 12 à base de fibres intissées.

Le matériau en feuille 12 est enduit sur une face interne 12a destinée à être au contact de la peau d'une membrane barrière 14 adhérente au matériau en feuille, à base de polymère de silicone réticulé, et d'une couche extérieure 16 de gel adhésif de silicone atraumatique et biocompatible.

Le matériau en feuille 12 est également enduit, sur une face externe 12b, d'une couche 18 d'un polymère anti-adhérent et déperlant à l'eau à base d'un polymère de fluorosilicone ou de fluorocarbone. Ce revêtement anti-adhérent 18 constitue la face externe du ruban 1 et présente un comportement de déperlance à l'eau afin d'éviter le mouillage de la nappe intissé lors des contacts du patient à l'eau. Elle permet enfin d'éviter que le ruban enroulé sous la forme d'un petit rouleau adhère à lui-même.

La nappe intissée 12 est constituée d'une structure continue de fibres, soit en une couche homogène, soit en deux couches homogènes de fibres interpénétrées comme cela est illustré.

Dans ce second cas, les couches confèrent aux deux faces de la nappe intissée 12, et donc du ruban final 1, des propriétés fonctionnelles bien distinctes.

En particulier, la face externe 12b est constituée de fibres denses et plus courtes pour lui conférer des propriétés de planéité, de douceur et anti-adhérentes. La face interne 12a est constituée quant à elle de fibres plus longues, moins densifiées, et de structure de surface plus rugueuse nécessaire à un ancrage mécanique et à un mouillage important des fibres lors de l'enduction du polymère de silicone constituant la membrane silicone intérieure.

La nappe intissée 12 est fabriquée par exemple selon un processus aqueux par voie papetière appelé « wet laid process». Les fibres sont constituées d'un mélange homogène de fibres courtes de cellulose et de fibres de longueur moyenne de polyéthylène téréphtalate (PET).

Les fibres cellulosiques confèrent à la nappe 12 les propriétés de remplissage et de planéité nécessaire pour une enduction régulière, associée à une faible résistance mécanique à la déchirure pour une déchirure aisée du ruban 1 à la main.

Les fibres polyéthylène téréphtalate confèrent quant à elle à la nappe 12 des propriétés mécanique suffisante aussi bien pour un maintien de la nappe 12 lors du processus d'enduction et de découpe de ruban 1 que pour la manipulation dudit ruban 1, par exemple par une infirmière, sans risque de rupture. Ces fibres polyéthylène permettent également une bonne résistance mécanique de la nappe 12 à l'état mouillé, ainsi qu'une bonne protection mécanique du patient contre tout risque de perforation ou d'abrasion.

Les fibres de cellulose et de PET sont généralement liées chimiquement entre elles à l'aide d'un agent chimique de liage en phase aqueuse, ou bien par thermofusion partielle à l'aide de cylindres à chaud sous pression. L'agent chimique de liaison est typiquement à base de polymères en émulsion couramment utilisé dans l'industrie papetière.

Avantageusement, le ratio de répartition entre les fibres varie de 10 à 40 % de PET pour 90 à 60 % de cellulose. Une gamme typique de poids de nappe intissée 12 varie de l'ordre de 30 à 70g/m² pour une épaisseur de 0,10 à 0,30 mm. Les fibres de cellulose présentent des fibres de longueur de l'ordre de 1mm à 20mm tandis que les fibres de PET présentent une structure cylindrique de masse linéique comprise entre environ 0.1dtex et 5dtex et de longueur de l'ordre de 2mm à 20mm.

Les fibres sont typiquement distribuées en couche simple homogène. Pour cette innovation, il a été développé une nappe en deux couches 13a et 13b de deux densités spécifiques de fibres qui procurent au produit final des propriétés fonctionnelles remarquables.

La couche extérieure 13b est constituée d'une haute densité de fibres. Les fonctions résultantes sont une bonne étanchéité au pré-polymère liquide de silicone constituant la membrane, pendant le processus d'enduction de cette membrane, ce qui permet d'éviter un transfert de ce polymère liquide vers la face externe 12b de la nappe 12. De plus, cela confère à la face externe 12b de la nappe 12, et donc du ruban final 1, un touché très souple de type velouté au contact du patient ou d'un vêtement qui recouvre la peau.

Afin d'augmenter encore cet aspect velouté, il est procédé en fin de fabrication de la nappe 12 à une opération d'enduction de la face externe 12b à l'aide d'une polymère anti-adhérant dont la fonctionnalité est de former la couche 18 permettant d'éviter que le gel silicone adhésif de la face intérieure 12a ne colle sur la face extérieure 12b lors des enroulements/déroulements du ruban 1, associée à des propriétés de faible mouillage lors du contact du pansement à l'eau, par exemple lors d'une douche ou du lavage du patient.

Typiquement, les agents anti-adhérant utilisés contre le collage du gel silicone adhésif et contre le mouillage par l'eau sont du type polymères polyfluorocarbonés ou fluorosilicones, présentés en émulsion aqueuse. Ils sont déposés sur la face externe 12b de la nappe 12 par tout type de procédé d'enduction rencontré typiquement dans l'industrie textile ou papetière par exemple à l'aide de cylindres ou par foulardage.

Lors de l'opération de séchage de l'eau de l'émulsion, un calandrage à chaud à l'aide de cylindre métallique poli-miroir peut encore parfaire cet aspect de surface.

La couche intérieure 13a est constituée quant à elle d'un mélange de fibres à plus basse densité de façon à procurer à la surface interne 12a une surface plus rugueuse, destinée à offrir un plus grand contact et un meilleur ancrage mécanique du polymère de silicone, associée à une meilleure liaison chimique entre le silicone et les fibres, notamment de PET.

Les couches 13a et 13b sont fabriquées ensemble à partir d'une même pulpe de dispersion de fibres, comprenant l'agent chimique de liaison des fibres, avec la possibilité de faire varier la concentration de pulpe au cours d'un processus de dépôt humide juste avant l'essorage de la pulpe et l'application de cylindres essoreurs à chaud de cette couche de pulpe puis séchage par air chaud de la nappe de fibres ainsi obtenue.

L'agent de liaison chimique des fibres doit être judicieusement choisi de façon à ne pas présenter d'interférence négative avec la polymérisation à catalyse platine du polymère constituant la membrane intérieure de silicone. Typiquement, on pourra choisir une émulsion de polymères acrylate, d'alcool polyvinylique, de polyamide ou de polyester.

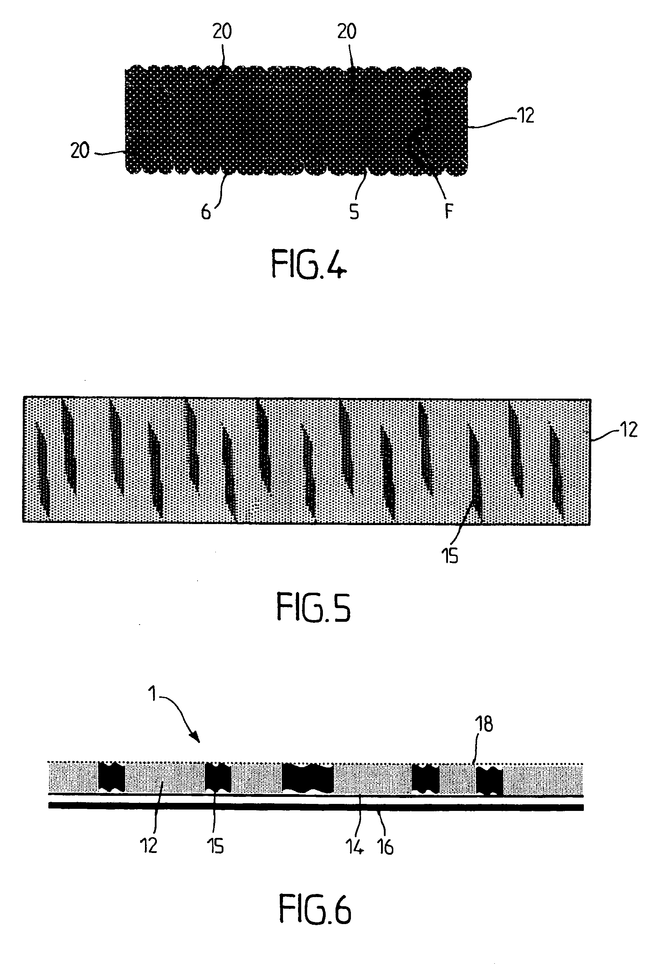

Une telle nappe intissée 12 présente ainsi une surface plane dont les fibres sont jointives et dont l'allongement sous contrainte de déformation est faible. Ce type de structure présente l'inconvénient de mal s'adapter à la fixation sur des parties du corps soumis à une grande déformation, telles que dans les plis ou les articulations. Pour améliorer le confort de pose et de port par le patient, et afin d'augmenter la sécurité de fixation sur la peau, il a été développé un ruban adhésif gel sur la base d'une nappe intissée 12 de fibres telles que décrit ci-dessus, qui reçoit après fabrication de la nappe une opération supplémentaire de froissement transversal et de gavage longitudinal de la matière en petits plis transversaux 15 donnant un aspect de « papier crépon », comme illustré sur les figure 5 et 6.

Cette opération de structuration longitudinale de la nappe intissée 12, qui engendre une suralimentation de la matière, permet d'obtenir des propriétés de déformation longitudinale de la nappe lui procurant une bonne capacité de déformation sous contrainte. Ainsi, l'allongement sous charge de cette structure est de l'ordre de 10 % sous une charge de 1N/cm et l'allongement avant rupture est de 20 %, contre 10 % pour une nappe intissée dépourvue de crêpage.

Cette opération est réalisée en dynamique par suralimentation, d'une longueur de nappe au travers d'un premier cylindre rotatif sur lequel est posée une racle métallique qui vient pincer la nappe défilante. L'excès de matière suralimenté par le premier cylindre ne pouvant passer entre la racle, un deuxième cylindre crée des petits plis transversaux 15 constituant une réserve de longueur pour de plus fort allongements sous contrainte, comme expliqué précédemment.

Il est également important d'éviter le traversement du silicone gel adhésif 16 au travers de la couche de fibres 12 lorsqu'une pression est appliquée sur le ruban par l'infirmière ou par le patient, pendant la pose du pansement ou du dispositif, ou bien préalablement à la pose par application de pression pendant la découpe et l'enroulement du rouleau de ruban.

De plus, il est également important d'éviter toute délamination de l'adhésif gel 16 lors du retrait du ruban 1 de la peau par arrachage.

Afin d'arriver à ce résultat, il convient à cet effet d'obtenir une forte adhésion entre le silicone gel adhésif 16 et la nappe d'intissé 12. Ainsi, un simple ancrage mécanique de la couche de silicone gel 16 sur les fibres 12 ne procure pas une sécurité suffisante au patient, et il est nécessaire d'améliorer l'adhésion mécanique par une adhésion d'ordre chimique entre la couche de la de silicone gel adhésif 16et la couche de fibres 12.

La présente innovation consiste à générer une membrane intermédiaire continue 14 faite d'un polymère de silicone réactif chimiquement de façon à créer une membrane barrière pour le traversement du gel adhésif 16 au travers des fibres 12, et à créer une adhésion mécanique et chimique forte avec les fibres 12 d'un coté et avec la couche de gel adhésif 16 de l'autre coté afin d'éviter le traversement du gel au travers des fibres et les arrachements et résidus de matière lors du retrait de la peau du ruban.

La membrane intermédiaire 14 est composée d'un mélange de pré-polymères linéaires ou partiellement branchés ou en structure tridimensionnelle de type résine, de haut poids moléculaire de type alkenyl ou vinyl silicone formulés avec un agent de liaison chimique de type organosilane et un système catalytique typiquement à base de alkyl-orthotitanate et dérivés du platine. La formulation réactive est réalisé en ligne par l'intermédiaire d'un équipement de pompage, dosage et de mélangeage dynamique ou statique en continu, et ainsi alimenter la ligne d'enduction en continu. Les produits typiques utilisés pour cette application peuvent être le SILBIONE HC2 2512 de la société Blue Star Silicone ou équivalent.

La ligne d'enduction est constituée d'un dispositif de laminage sur cylindre métallique, d'un film liquide de type cylindre et racle, suivi d'un système de transfert du film liquide sur la face interne 12a de la nappe de fibres 12 du type reverse roll ou cylindre en rotation à l'envers soit en co-rotation, soit en contra-rotation, soit cylindre en l'air.

Ensuite, la nappe intissée 12 enduite du film liquide de pré-polymères et du système catalytique est transférée en continue au travers d'un four à air chaud afin d'activer la polymérisation par une réaction de polyaddition des pré-polymères pour obtenir en sortie de four une couche solide de silicone polymérisé formant une membrane continue 14 et, de façon conjointe, une liaison chimique entre les fibres de polyéthylène téréphtalate 12 et le polymère de silicone formant la membrane 14 qui va générer l'adhérence forte entre le gel silicone adhésif 16 enduit dans une deuxième étape et la nappe de fibres de l'intissé 12.

Les poids typiques déposés pour la constitution de cette membrane barrière 14 sont de 10 à 70g/m², dépendant de la structure et de la surface de la face interne 12a de la nappe intissé 12.

La couche de silicone gel adhésif 16 réalisée dans un deuxième temps sur la face interne 12a de la nappe intissée 12 est constituée d'un mélange de pré-polymères de silicone de type alkenyl ou vinyl silicone de bas poids moléculaire nécessaires à l'obtention d'une structure réticulé de type « gel », mélangé avec des pré-polymères de type hydrogéno-siloxane et d'un système de catalyse à base de platine et de régulateurs de cinétique de polymérisation de type polyols, de façon à obtenir un mélange final, dont la durée de vie en pot (pot life) soit compatible avec l'alimentation continue de la ligne d'enduction de la couche de gel et de nature à créer une copolymérisation avec la première couche constituant la membrane barrière 14.

Le type de silicone utilisé est un polymère de silicone gel réticulé comportant des propriétés atraumatique, une totale biocompatibilité au contact de la peau et de la plaie, enduit sur une membrane de silicone 14 fortement adhérée sur la face interne 12a des fibres de la nappe intissé 12.

Les poids de silicone déposé pour cette couche de gel adhésif 16 sont typiquement dans une fourchette de 50 à 200 g/m² selon le niveau d'adhérence souhaité sur peau pour le ruban adhésif.

Les produits silicone traditionnellement utilisés pour ce dépôt peuvent être le SILBIONE HC 2 2030 de la société Blue Star Silicone ou bien équivalent.

La perméabilité à la vapeur d'eau de ce matériau peut aller de 500 à 2000 g/m²/24h mesurée à l'eau selon la méthode de la coupe inversée, ce qui est un niveau de perméabilité considéré comme suffisante sur peau saine pour éviter les phénomènes de macération en milieu fermé due à l'exsudation.

Une couche d'enduction discontinue peut également être réalisée par différente technologie d'enduction notamment la flexogravure par cylindres, l'utilisation de buses de coulée continue, de cylindres à écran métallique perforé ou bien encore par cylindre transfert. Le diamètre des surfaces non enduites sera d'une dimension faible, de l'ordre de 0,1 à 0,5 mm, de façon à conserver une force suffisante d'adhésion du gel sur la peau. Une telle couche d'enduction discontinue 16 présente un intérêt pour augmenter la perméabilité à la vapeur résultante de l'exsudation de la peau au travers du ruban à des niveaux de perméabilité supérieure à 1000 g/m²/24h et ainsi éviter tout risque de macération de la peau en cas de plaie fortement exsudante.

Il est également possible d'obtenir une présentation coloré du ruban adhésif 1 à l'aide de différentes méthodes.

Selon une première technique de pigmentation de la couche de silicone barrière 14 ou de la couche de silicone gel 16, on utilise une pré-dispersion de pigment dans une huile réactive à base de pré-polymère réactif de silicone qui sera co-polymérisé en même temps que les pré-polymères de silicone constituant la couche barrière 14 ou la couche de gel 16.

Selon une autre technique de teinture des fibres de la nappe intissée, on utilise les procédés traditionnels de teinture dans la masse des fibres textiles ou la pigmentation du polymère de liage des fibres introduit dans la pulpe aqueuse de fibres lors de la fabrication de la nappe intissée 12.

Bien évidemment, le processus de fabrication décrit ci dessus sera également utilisé, pour l'enduction de support à base de textile destiné à réaliser des rubans adhésifs silicone gel.

Après polymérisation de la couche de gel adhésive de silicone 16, la feuille du matériau ainsi fabriqué sera enroulé sur elle même une face sur l'autre face afin d'être découpée en rouleau de ruban de largeur finale pour l'usage final.

Avant enroulement, il peut être procédé à la perforation de la surface du matériau, à l'aide de cylindre à aiguilles chaude ou bien à l'aide de cylindre-poinçons de découpe sous pression, de façon à créer des petites perforations 20 (figure 4) de 0,05 à 0,5 mm de diamètre de manière à obtenir une structure perforée de très grande perméabilité à la vapeur supérieure à 2000 g/m²/24h ou bien pour constituer la base d'un matériau pour pansement laissant traverser les exsudats liquide de la plaie qui seront absorbés ensuite sur la face supérieure par un matériau absorbant.

Comme cela est illustré sur les figures 3 à 5, les bords 5 du ruban 1 enroulé en rouleau pourront également être munis de petites entailles 6 de forme diamant ou demi disque découpées périodiquement dans la longueur de façon à créer des concentrations de contrainte de déchirure. Ainsi, la propagation de la déchirure transversale F du ruban 1 facilitera la découpe manuelle d'un morceau de ruban par l'usager final (patient, personnel médical).

Il va de soi que la description détaillée de l'objet de l'Invention, donnée uniquement à titre d'illustration, ne constitue en aucune manière une limitation, les équivalents techniques étant également compris dans le champ de la présente invention.

## Revendications

1. Ruban adhésif (1) en silicone destiné à des applications médicales, comprenant un matériau en feuille (12) à base de fibres intissées, enduit sur une face interne (12a) destinée à être au contact de la peau, d'une membrane barrière (14) adhérente au matériau en feuille, à base de polymère de silicone réticulé et d'une couche (16) de gel adhésif de silicone atraumatique et biocompatible, **caractérisé en ce que** qu'il est enduit, sur une face externe (12b), d'une couche (18) d'un polymère anti-adhérent et déperlant à l'eau à base d'un polymère de fluorosilicone ou de fluorocarbone.

2. Ruban selon la revendication 1, **caractérisé en ce que** le matériau en feuille (12) est composé d'une ou deux nappes (13a ; 13b) d'un mélange de fibres à faible résistance à la déchirure et aisément déchirable à la main, fabriquée(s) à partir d'un mélange de fibres courtes de cellulose présentent une longueur de l'ordre de 1mm à 20mm et de fibres de polyéthylène téréphtalate de longueur moyenne présentent une longueur de l'ordre de 2mm à 20mm, collées entre elles par un calandrage sous pression à chaud et/ou par un agent chimique de liage en émulsion aqueuse.

3. Ruban selon la revendication 2, **caractérisé en ce que**
- le ratio de partage entre les fibres varie de 10 à 40% de PET pour 90 à 60% de cellulose,
- le poids de la nappe intissée varie de l'ordre de 30 à 70g/m2 pour une épaisseur de 0,10 à 0,30 mm, et
- les fibres de PET présentent des structures de fibre cylindrique de masse linéique comprise entre 0.1dtex et 5dtex.

4. Ruban selon la revendication 3, **caractérisé en ce que** la nappe intissée(12) présente une structure de surface comportant des petits plis (15) de matière en surabondance dans le sens transversal de façon à conférer au matériau des propriétés de déformation sous faible contrainte d'allongement dans le sens longitudinal.

5. Ruban selon la revendication 4, **caractérisé en ce qu'il** comporte en outre une fine membrane barrière continue ou une couche discontinue en polymère réticulé de silicone (14) contrecollée aux fibres de la face interne (12a) de la nappe intissée (12).

6. Ruban selon la revendication 5, **caractérisé en ce qu'il** comporte également une couche (16) d'un gel de silicone réticulé adhésif atraumatique et biocompatible pour la peau adhérent par réaction de copolymérisation sur la membrane en silicone réticulée formant barrière sur la face interne de la nappe intissée.

7. Ruban selon l'une quelconque des revendications précédentes, **caractérisé en ce qu'il** est perforé de petits orifices (20) de façon à lui procurer une grande perméabilité à la vapeur issue de l'exsudation de la peau et afin d'éviter une macération de la peau humide.

8. Ruban selon l'une quelconque des revendications précédentes, **caractérisé en ce que** la feuille de fibres intissées est perforée d'orifices (20) de diamètre de 0,05 à 0,5 mm de manière à procurer au matériau final un aspect de structure textile tissé ou tricoté afin d'améliorer la perméabilité à la vapeur d'eau issue de l'exsudation de la peau, d'améliorer la déchirabilité, et/ou de procurer à la surface externe du matériau des propriétés de faible friction et de grand confort de port, au contact avec tout textile ou vêtement, et/ou afin de procurer pour le patient un grand confort de port, au contact de zone de la peau susceptible de se déformer, de se plier, ou de comporter des zones creuses et des zones à relief.

9. Ruban selon l'une quelconque des revendications précédentes, **caractérisé en ce que** ses bords (5) sont découpés sur toute leur longueur selon un motif géométrique répétitif (6), tel que dents triangulaires, des demi disques, ou des indentations aléatoires, afin de procurer au ruban (1) une facilité de déchirure manuelle transversale (F).

10. Ruban selon l'une quelconque des revendications précédentes, **caractérisé en ce que** la perméabilité à la vapeur d'eau de la couche de gel adhésif de silicone (16) est comprise entre environ 500 à 2000 g/m²/24h, mesurée à l'eau selon la méthode de la coupe inversée (norme EN 13 726-2), et de préférence d'environ 1000 g/m²/24h.

11. Ruban selon l'une quelconque des revendications précédentes, **caractérisé en ce que** les fibres intissées (12) sont teintes, et/ou l'agent chimique de liaison des fibres est pigmenté et enduit d'une couche barrière de silicone réticulé et d'une couche d'un adhésif de gel de silicone réticulé dans lesquelles les polymères de silicone sont pigmentés.

## Patentansprüche

1. Adhäsives Silikonband (1) für medizinische Anwendungen, umfassend ein Blattmaterial (12) auf Vliesfaserbasis, welches auf einer Innenseite (12a), welche bestimmt ist im Kontakt mit der Haut zu sein, mit einer an dem Blattmaterial anhaftenden Sperrmembran (14), basierend auf vernetztem Silikonpolymer, und mit einer Schicht aus adhäsivem Gel aus atraumatischem und biokompatiblem Silikon beschichtet ist, **dadurch gekennzeichnet, dass** es auf einer Außenseite (12b) mit einer Schicht (18) eines nicht-haftenden und wasserabweisenden Polymers, basierend auf einem Polymer aus Fluorsilikon oder Fluorkohlenstoff, beschichtet ist.

2. Band nach Anspruch 1, **dadurch gekennzeichnet, dass** das Blattmaterial (12) aus einer oder zwei Lage(n) (13a; 13b) einer Mischung aus Fasern mit niedrigem Reiß-Widerstand, und welche leicht mit den Händen zerreißbar ist, besteht, welche hergestellt wird/werden aus einer Mischung aus kurzen Cellulosefasern, die eine Länge im Bereich von 1 mm bis 20 mm aufweisen, und aus Polyethylenterephthalatfasern, die eine mittlere Länge im Bereich von 2mm bis 20mm aufweisen, welche durch Heißkalandrieren unter Druck und/oder durch ein chemisches Bindemittel in wässriger Emulsion miteinander verbunden sind.

3. Band nach Anspruch 2, **dadurch gekennzeichnet, dass**
- das Verteilungsverhältnis zwischen den Fasern von 10 bis 40% PET zu 90 bis 60 % Cellulose variiert,
- das Gewicht der Vlieslage im Bereich von 30 bis 70 g/m2 für eine Dicke von 0,10 bis 0,30 mm variiert, und
- die PET-Fasern Strukturen zylindrischer Faser mit einer linearen Dichte, die zwischen 0,1 dtex und 5 dtex umfasst ist, aufweisen.

4. Band nach Anspruch 3, **dadurch gekennzeichnet, dass** die Vlieslage (12) eine Oberflächenstruktur aufweist, welche kleine Falten (15) aus einem Material in Überfülle, in Querrichtung umfasst, so dass sie dem Material Verformungseigenschaften unter niedriger Dehnungsbelastung in der Längsrichtung verleiht.

5. Band nach Anspruch 4, **dadurch gekennzeichnet, dass** es zusätzlich eine kontinuierliche dünne Sperrmembran oder eine diskontinuierliche Schicht aus vernetztem Süikonpolymer (14) umfasst, welche auf die Fasern der Innenseite (12a) der Vlieslage (12) aufkaschiert ist.

6. Band nach Anspruch 5, **dadurch gekennzeichnet, dass** es ebenfalls eine Schicht (16) eines adhäsiven, für die Haut atraumatischen und biokompatiblen vernetzten Silikongels umfasst, welches durch Copolymerisationsreaktion an der vernetzten Silikonmembran haftet, welche als Sperre auf der Innenseite der Vlieslage dient.

7. Band nach einem der vorhergehenden Ansprüche, **dadurch gekennzeichnet, dass** es mit kleinen Löchern (20) perforiert ist, um ihm eine hohe Durchlässigkeit gegenüber Dampf aus der Ausschwitzung der Haut zu verleihen, und um die Mazeration der nassen Haut zu vermeiden.

8. Band nach einem der vorhergehenden Ansprüche, **dadurch gekennzeichnet, dass** das Vliesfaserblatt mit Löchern (20) mit einem Durchmesser von 0,05 bis 0,5 mm perforiert ist, um dem finalen Material einen Aspekt einer gewebten oder gestrickten Textilstruktur zu verleihen, um die Durchlässigkeit gegenüber Wasserdampf aus der Ausschwitzung der Haut zu verbessern, um die Reißfähigkeit zu verbessern, und / oder um der Außenfläche des Materials Eigenschaften von niedriger Reibung und von hohem Tragekomfort zu verleihen, wenn es im Kontakt mit einer Textil- oder Bekleidungsstück ist, und / oder um an den Patienten einen hohen Tragekomfort zu verleihen, wenn es im Kontakt mit Hautflächen ist, welche sich verformen, sich falten, oder welche vertiefte Bereiche und erhöhte Bereiche umfassen können.

9. Band nach einem der vorhergehenden Ansprüche, **dadurch gekennzeichnet, dass** seine Ränder (5) über ihre gesamte Länge gemäß einem wiederholenden geometrischen Muster (6) geschnitten werden, wie z.B dreieckigen Zahnungen, Halbscheiben, oder zufälligen Einschnitten, um dem Band (1) eine manuelle Querreißleichtigkeit (F) zu verleihen,

10. Band nach einem der vorhergehenden Ansprüche, **dadurch gekennzeichnet, dass** die Durchlässigkeit gegenüber Wasserdampf der adhäsiven Silikongelschicht (16) zwischen 500 und 2000 g/m²/24h umfasst ist, gemessen mit Wasser nach dem "Inverted Cup" Verfahren (Norm EN 13 726-2), vorzugsweise etwa 1000 g/m²/24h.

11. Band nach einem der vorhergehenden Ansprüche, **dadurch gekennzeichnet, dass** die Vliesfasern (12) gefärbt sind und/oder dass das chemische Bindemittel der Fasern pigmentiert ist und mit einer Sperrschicht aus vernetztem Silikon und mit einem adhäsiven vernetzen Silikongel beschichtet ist, in denen die Silikonpolymere pigmentiert sind.

## Claims

1. Adhesive tape (1) made of silicone for medical applications, comprising a sheet material (12) based on nonwoven fibres which is coated, on an inside face (12a) that is to be in contact with the skin, with a barrier membrane (14) which adheres to the sheet material and is based on crosslinked silicone polymer, and with a layer (16) of atraumatic and biocompatible adhesive silicone gel, **characterised in that** it is coated on an outside face (12b) with a layer (18) of a non-stick and water-repellent polymer based on a fluorosilicone or fluorocarbon polymer.

2. Tape according to claim 1, **characterised in that** the sheet material (12) is composed of one or two layers (13a; 13b) of a mixture of fibres which have low tear strength and are easily tearable by hand, manufactured from a mixture of short cellulose fibres having a length of approximately from 1 mm to 20 mm and polyethylene terephthalate fibres of medium length having a length of approximately from 2 mm to 20 mm, which fibres are bonded together by hot calendering under pressure and/or by a chemical bonding agent in aqueous emulsion.

3. Tape according to claim 2, **characterised in that**
- the partition ratio between the fibres varies from 10 to 40% PET for 90 to 60% cellulose,
- the weight of the nonwoven layer varies from approximately 30 to 70 g/m² for a thickness of from 0.10 to 0.30 mm, and
- the PET fibres have cylindrical fibre structures of linear density from 0.1 dtex to 5 dtex.

4. Tape according to claim 3, **characterised in that** the nonwoven layer (12) has a surface structure comprising a large number of small folds (15) of super abundant material in the transverse direction in order to impart to the material properties of deformation under low elongation stress in the longitudinal direction.

5. Tape according to claim 4, **characterised in that** it further comprises a continuous fine barrier membrane or a discontinuous layer of crosslinked silicone polymer (4) laminated to the fibres of the inside face (12a) of the nonwoven layer (12).

6. Tape according to claim 5, **characterised in that** it also comprises a layer (16) of a crosslinked silicone adhesive gel which is atraumatic and biocompatible for the skin, which layer adheres by a copolymerisation reaction to the crosslinked silicone membrane forming a barrier on the inside face of the nonwoven layer.

7. Tape according to any one of the preceding claims, **characterised in that** it is perforated with small orifices (20) in order to provide it with high permeability to vapour resulting from the exudation of the skin and in order to avoid laceration of wet skin.

8. Tape according to any one of the preceding claims, **characterised in that** the sheet of nonwoven fibres is perforated with orifices (20) having a diameter of from 0.05 to 0.5 mm in order to provide the final material with the appearance of a woven or knitted textile structure so as to improve the permeability to water vapour resulting from the exudation of the skin, to improve tearability, and/or to provide the outside surface of the material with properties of low friction and high wearing comfort, in contact with any textile or item of clothing, and/or in order to provide for the patient high wearing comfort in contact with an area of the skin which may deform, fold or include hollow areas and raised areas.

9. Tape according to any one of the preceding claims, **characterised in that** its edges (5) are cut over their entire length in a repetitive geometric pattern (6), such as triangular teeth, half-discs or random indentations, in order to make the tape (1) easy to tear transversely (F) by hand.

10. Tape according to any one of the preceding claims, **characterised in that** the water vapour permeability of the layer of adhesive silicone gel (16) is approximately from 500 to 2000 g/m²/24h, measured with water according to the inverted cup method (standard EN 13 726-2), and preferably approximately 1000 g/m²/24h.

11. Tape according to any one of the preceding claims, **characterised in that** the nonwoven fibres (12) are dyed, and/or the chemical agent for bonding the fibres is pigmented and coated with a barrier layer of crosslinked silicone and with a layer of a crosslinked silicone gel adhesive, in which layers the silicone polymers are pigmented.
